(19) **Europäisches Patentamt / European Patent Office / Office européen des brevets**

(11) **EP 4 736 849 A1**

(12) **EUROPEAN PATENT APPLICATION**
published in accordance with Art. 153(4) EPC

(43) Date of publication:
06.05.2026 Bulletin 2026/19

(21) Application number: 24831533.5

(22) Date of filing: 27.05.2024

(51) International Patent Classification (IPC):
$A61K\ 31/16^{(2006.01)}$   $A23L\ 33/10^{(2016.01)}$
$A23L\ 33/15^{(2016.01)}$   $A23L\ 33/17^{(2016.01)}$
$A23L\ 33/18^{(2016.01)}$   $A23L\ 33/105^{(2016.01)}$
$A23L\ 33/115^{(2016.01)}$   $A23L\ 33/175^{(2016.01)}$
$A61K\ 8/42^{(2006.01)}$   $A61K\ 8/44^{(2006.01)}$
$A61K\ 8/46^{(2006.01)}$   $A61K\ 8/64^{(2006.01)}$
$A61K\ 8/9789^{(2017.01)}$   $A61K\ 8/9794^{(2017.01)}$
$A61K\ 31/047^{(2006.01)}$

(52) Cooperative Patent Classification (CPC):
A23L 33/10; A23L 33/105; A23L 33/115;
A23L 33/15; A23L 33/17; A23L 33/175;
A23L 33/18; A61K 8/42; A61K 8/44; A61K 8/46;
A61K 8/64; A61K 8/9789; A61K 8/9794;
A61K 31/047; A61K 31/16;        (Cont.)

(86) International application number:
PCT/JP2024/019454

(87) International publication number:
WO 2025/004662 (02.01.2025 Gazette 2025/01)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 29.06.2023  JP 2023107296
29.06.2023  JP 2023107297
24.08.2023  JP 2023136557
20.11.2023  JP 2023196515

(71) Applicant: SUNSTAR INC.
Takatsuki-shi, Osaka 569-1195 (JP)

(72) Inventors:
• REHAB, Alshargabi
Takatsuki-shi, Osaka 569-1195 (JP)
• ISHIKADO, Atsushi
Takatsuki-shi, Osaka 569-1195 (JP)
• MATSUMOTO, Motonobu
Takatsuki-shi, Osaka 569-1195 (JP)

(74) Representative: Müller-Boré & Partner
Patentanwälte PartG mbB
Friedenheimer Brücke 21
80639 München (DE)

(54) **COMPOSITION FOR ACTIVATING MITOCHONDRIA**

(57)   Provided is a method that can activate metabolism. Specifically provided is a composition for activating mitochondria, comprising a specific component.

Fig. 4

**(Cont. next page)**

(52) Cooperative Patent Classification (CPC): (Cont.)
**A61K 31/197; A61K 31/198; A61K 31/7032;
A61K 36/258; A61K 36/28; A61K 36/355;
A61K 36/46; A61K 36/53; A61K 36/54;
A61K 36/63; A61K 36/64; A61K 36/65;
A61K 36/82; A61K 36/899; A61K 36/9068;
A61K 38/17; A61P 1/02; A61P 3/00; A61P 3/04;
A61P 3/10; A61P 9/00; A61P 13/12; A61P 19/10;
A61P 21/00; A61P 25/00; A61P 25/28;
A61P 27/02; A61P 43/00; A61Q 11/00;
C07K 14/46**

**Description**

Technical Field

The present disclosure relates to a composition for activating mitochondria and the like.

Background Art

Various factors (e.g., high-glucose loads) are known to increase oxidative stress within cells, causing metabolic abnormalities. In particular, diabetic patients and those at risk of developing diabetes are considered to receive high-glucose loads, which lead to metabolic abnormalities, triggering various symptoms. These symptoms may also include symptoms in the oral cavity.

For example, it has been reported that the metabolic function of periodontal tissues is impaired in diabetic conditions, and that activating the metabolic function of the gingiva of diabetic rats inhibits alveolar bone resorption.

Citation List

Non-patent Literature

NPL 1: J Clin Periodontol 2017; 44: 463-471. IADR/PER General Session 2018; Presentation ID 1622

Summary of Invention

Technical Problem

The present inventors searched for a means capable of activating metabolism.

Solution to Problem

For example, even if oxidative stress within cells increases, metabolism activation can be expected to cause fewer metabolic abnormalities. For this reason, a means capable of activating metabolism is considered to be useful.

The present inventors focused on the function of mitochondria, which play a significant role in intracellular metabolism, particularly the ability to produce ATP and/or consume oxygen in cells, and searched for materials that can enhance ATP production and the oxygen consumption rate, or improve reduction in ATP production and reduction in the oxygen consumption rate.

The present disclosure includes, for example, the subject matter set forth in the following items.

Item 1-1.

A composition for activating mitochondria, comprising at least one selected from the group consisting of components listed in the following table.

Table 1a

| Chamomile extract |
| --- |
| Cistanche extract |
| Olive leaf extract |
| Inositol |
| Eucommia extract |
| Arnica flower extract |
| Reduced palatinose |
| Honeysuckle leaf extract |
| Peony root extract |
| *Panax ginseng* root extract |

(continued)

| |
|---|
| Ginger extract |
| Placenta extract |
| *Lamium album* extract |
| Melilotus extract |
| Isoleucine |
| Willow extract |
| Wild grape extract |
| Valerian root extract |
| Fennel extract |
| Devil's claw extract |
| Hypericum extract |
| Cholecalciferol |
| Glycyrrhizic acid or a salt thereof |
| Senega extract |
| Allantoin |
| Rice bran extract |
| Fermented black garlic extract |
| Alanine |
| Purple brown rice extract |
| Protamine or a salt thereof |
| Wild thyme extract |
| Cinnamaldehyde |
| Turmeric rhizome extract |
| *Panax notoginseng* extract |
| Red ginger extract |
| Paramylon |
| Oat extract |
| Plantago herb extract |
| Cyclodextrin |
| *Houttuynia cordata* leaf extract |
| Arginine |
| Goji berry extract |
| Ceramide |
| Methionine |
| Rosemary leaf extract |
| Echinacea extract |
| Yacon leaf extract |
| Pantothenic acid |
| Panthenol |
| Soybean extract |

Item 1-2.

The composition according to Item 1-1, wherein the mitochondria are mitochondria in periodontal ligament cells.

Item 1-3.

The composition according to Item 1-1 or 1-2, wherein mitochondrial activation is an increase in oxygen consumption in mitochondria.

Item 2-1.

A composition for activating mitochondria, comprising at least one selected from the group consisting of pantothenic acid or a salt thereof, protamine or a salt thereof, and methionine or a salt thereof.

Item 2-2.

The composition according to Item 2-1, wherein the mitochondria are mitochondria in periodontal ligament cells.

Item 2-3.

The composition according to Item 2-1 or 2-2, wherein mitochondrial activation is at least one selected from the group consisting of an increase in oxygen consumption in mitochondria, an improvement in ATP production capacity in mitochondria, and an increase in mitochondria.

Item 2-4.

The composition according to any one of Items 2-1 to 2-3, which is used to improve periodontal disease in a diabetic patient.

Item 2-5.

A composition for improving a symptom caused by diabetes, prediabetes, or an age-related metabolic disorder, the composition comprising at least one selected from the group consisting of pantothenic acid or a salt thereof, protamine or a salt thereof, and methionine or a salt thereof.

Item 2-6.

The composition according to Item 2-5, wherein the symptom caused by diabetes, prediabetes, or an age-related metabolic disorder is obesity, renal dysfunction, retinopathy, neuropathy, cardiovascular disease, brain dysfunction, dementia, frailty, muscle weakness, or osteoporosis.

Item 2-7.

The composition according to any one of Items 2-1 to 2-6, which is an oral composition or a composition for oral cavity.

Item 2-8.

The composition according to any one of Items 2-1 to 2-6, which is a food composition.

Item 2-9.

The composition for oral cavity according to Item 2-7, which is an ointment, a paste, a dermatological paste, a gel, a liquid, a spray, a mouthwash, a liquid dentifrice, a toothpaste, or a gum.

Item 3-1.

A composition for activating mitochondria, comprising:

(A) pantothenic acid or a salt thereof, and
(B) at least one selected from the group consisting of methionine or a salt thereof, protamine or a salt thereof, ceramide, and rice bran.

Item 3-2.

The composition according to Item 3-1, wherein the mitochondria are mitochondria in periodontal ligament cells.

Item 3-3.

A composition for improving periodontal disease, comprising:

(A) pantothenic acid or a salt thereof, and
(B) at least one selected from the group consisting of methionine or a salt thereof, protamine or a salt thereof, ceramide, and rice bran.

Item 3-4.

The composition according to Item 3-3, which is used to improve periodontal disease in a diabetic patient.

Item 3-5.

A composition for improving a symptom caused by diabetes or an age-related metabolic disorder, comprising:

(A) pantothenic acid or a salt thereof, and
(B) at least one selected from the group consisting of methionine or a salt thereof, protamine or a salt thereof, ceramide, and rice bran.

Item 3-6.

The composition according to Item 3-5, wherein the symptom caused by diabetes or an age-related metabolic disorder is obesity, renal dysfunction, retinopathy, neuropathy, cardiovascular disease, brain dysfunction, dementia, frailty, muscle weakness, or osteoporosis.

Item 3-7.

The composition according to any one of Items 3-1 to 3-6, which is an oral composition.

Item 3-8.

The composition according to Item 3-7, which is a food composition.

Item 4-1.

A composition for activating mitochondria, comprising:

(C) panthenol, and
(D) at least one selected from the group consisting of cinnamon oil, and glutamic acid or a salt thereof.

Item 4-2.

The composition according to Item 4-1, wherein the mitochondria are mitochondria in periodontal ligament cells.

Item 4-3.

A composition for improving periodontal disease, comprising:

(C) panthenol, and

(D) at least one selected from the group consisting of cinnamon oil, and glutamic acid or a salt thereof.

Item 4-4.

The composition according to Item 4-3, which is used to improve periodontal disease in a diabetic patient.

Item 4-5.

The composition according to any one of Items 4-1 to 4-4, which is a composition for oral cavity.

Item A-1.

A composition for activating mitochondria, comprising at least one selected from the group consisting of:

panthenol,
pantothenic acid or a salt thereof,
protamine or a salt thereof, and
methionine or a salt thereof.

Item A-2.

The composition for activating mitochondria according to Item A-1, comprising:

(A) pantothenic acid or a salt thereof, and
(B) at least one selected from the group consisting of methionine or a salt thereof, protamine or a salt thereof, ceramide, and rice bran.

Item A-3.

The composition for activating mitochondria according to Item A-1, comprising:

(C) panthenol, and
(D) at least one selected from the group consisting of cinnamon oil, and glutamic acid or a salt thereof.

Item A-4.

The composition according to any one of Items A-1 to A-3, wherein the mitochondria are mitochondria in periodontal ligament cells.

Item B-1.

A composition for improving periodontal disease, comprising at least one selected from the group consisting of:

panthenol,
pantothenic acid or a salt thereof,
protamine or a salt thereof, and
methionine or a salt thereof.

Item B-2.

The composition for improving periodontal disease according to Item B-1 comprising:

(A) pantothenic acid or a salt thereof, and
(B) at least one selected from the group consisting of methionine or a salt thereof, protamine or a salt thereof, ceramide, and rice bran.

Item B-3.

The composition for improving periodontal disease according to Item B-1, comprising:

(C) panthenol, and
(D) at least one selected from the group consisting of cinnamon oil, and glutamic acid or a salt thereof.

Item B-4.

The composition according to any one of Items B-1 to B-3, which is used to improve periodontal disease in a diabetic patient.

Item C-1.

A composition for improving a symptom caused by diabetes, prediabetes, or an age-related metabolic disorder, the composition comprising at least one selected from the group consisting of:

panthenol,
pantothenic acid or a salt thereof,
protamine or a salt thereof, and
methionine or a salt thereof.

Item C-2.

The composition according to Item C-1, comprising:

(A) pantothenic acid or a salt thereof, and
(B) at least one selected from the group consisting of methionine or a salt thereof, protamine or a salt thereof, ceramide, and rice bran.

Item C-3.

The composition for improving periodontal disease according to Item C-1, comprising:

(C) panthenol, and
(D) at least one selected from the group consisting of cinnamon oil, and glutamic acid or a salt thereof.

Item C-4.

The composition according to any one of Items C-1 to C-3, wherein the symptom caused by diabetes, prediabetes, or an age-related metabolic disorder is obesity, renal dysfunction, retinopathy, neuropathy, cardiovascular disease, brain dysfunction, dementia, frailty, muscle weakness, or osteoporosis.

Item C-5.

The composition according to any one of Items A-1 to A-4, B-1 to B-4, and C-1 to C-4, which is an oral composition or a composition for oral cavity.

Item C-6.

The composition according to any one of Items A-1 to A-4, B-1 to B-4, and C-1 to C-4, which is a food composition.

Item C-7.

The composition for oral cavity according to Item C-5, which is an ointment, a paste, a dermatological paste, a gel, a liquid, a spray, a mouthwash, a liquid dentifrice, a toothpaste, or a gum.

Item A-i.

A compound for use in activating mitochondria, the compound being at least one compound selected from the group consisting of:

panthenol,
pantothenic acid or a salt thereof,
protamine or a salt thereof, and
methionine or a salt thereof.

Item A-ii.

The compound for use in activating mitochondria according to Item A-i, wherein the compound comprises:

(A) pantothenic acid or a salt thereof, and
(B) at least one selected from the group consisting of methionine or a salt thereof, protamine or a salt thereof, ceramide, and rice bran.

Item A-iii.

The compound for use in activating mitochondria according to Item A-i, wherein the compound comprises:

(C) panthenol, and
(D) at least one selected from the group consisting of cinnamon oil, and glutamic acid or a salt thereof.

Item A-iv.

The compound for use in activating mitochondria according to any one of Items A-i to A-iii, wherein the mitochondria are mitochondria in periodontal ligament cells.

Item B-i.

A compound for use in improving periodontal disease, the compound being at least one compound selected from the group consisting of:

panthenol,
pantothenic acid or a salt thereof,
protamine or a salt thereof, and
methionine or a salt thereof.

Item B-ii.

The compound for use in improving periodontal disease according to Item B-i, wherein the compound comprises:

(A) pantothenic acid or a salt thereof, and
(B) at least one selected from the group consisting of methionine or a salt thereof, protamine or a salt thereof, ceramide, and rice bran.

Item B-iii.

The compound for use in improving periodontal disease according to Item B-i, wherein the compound comprises:

(C) panthenol, and
(D) at least one selected from the group consisting of cinnamon oil, and glutamic acid or a salt thereof.

Item B-iv.

The compound for use in improving periodontal disease according to any one of Items B-i to B-iii, wherein the periodontal disease is periodontal disease in a diabetic patient.

Item C-i.

A compound for use in improving a symptom caused by diabetes, prediabetes, or an age-related metabolic disorder, the compound being at least one compound selected from the group consisting of:

> panthenol,
> pantothenic acid or a salt thereof,
> protamine or a salt thereof, and
> methionine or a salt thereof.

Item C-ii.

The compound for use in improving a symptom caused by diabetes, prediabetes, or an age-related metabolic disorder according to Item C-i, wherein the compound comprises:

> (A) pantothenic acid or a salt thereof, and
> (B) at least one selected from the group consisting of methionine or a salt thereof, protamine or a salt thereof, ceramide, and rice bran.

Item C-iii.

The compound for use in improving a symptom caused by diabetes, prediabetes, or an age-related metabolic disorder according to Item C-i, wherein the compound comprises:

> (C) panthenol, and
> (D) at least one selected from the group consisting of cinnamon oil, and glutamic acid or a salt thereof.

Item C-iv.

The compound for use in improving a symptom caused by diabetes, prediabetes, or an age-related metabolic disorder according to any one of Items C-i to C-iii, wherein the symptom caused by diabetes, prediabetes, or an age-related metabolic disorder is obesity, renal dysfunction, retinopathy, neuropathy, cardiovascular disease, brain dysfunction, dementia, frailty, muscle weakness, or osteoporosis.

Item C-v.

An oral composition or composition for oral cavity, comprising the compound according to any one of Items A-i to A-iv, B-i to B-iv, and C-i to C-iv.

Item C-vi.

A food composition, comprising the compound according to any one of Items A-i to A-iv, B-i to B-iv, and C-i to C-iv.

Item C-vii.

The composition for oral cavity according to Item C-v, which is an ointment, a paste, a dermatological paste, a gel, a liquid, a spray, a mouthwash, a liquid dentifrice, a toothpaste, or a gum.

Advantageous Effects of Invention

It is possible to activate mitochondria and improve metabolic functions related to mitochondria (particularly improve the ability to produce ATP and/or consume oxygen of mitochondria). For example, even if oxidative stress within cells increases, the occurrence of metabolic abnormalities can be prevented by improving metabolic functions.
Although not particularly limited, it is possible to improve metabolic abnormalities caused by high-glucose loads, particularly the decline in metabolic functions related to mitochondria. In particular, it is possible to improve reduction in ATP production and reduction in the oxygen consumption rate caused by high-glucose loads.

Furthermore, improving the mitochondrial metabolism of cells in the oral cavity (e.g., gingival cells and periodontal ligament cells) makes it possible to activate the metabolic functions of the cells in the oral cavity or suppress the decline in metabolic functions, which is expected to help maintain oral health (e.g., prevent periodontal disease and improve the symptoms of periodontal disease).

Brief Description of Drawings

Fig. 1a shows the ATP reduction improvement rate when treating human periodontal ligament fibroblasts with calcium pantothenate after high-glucose treatment.
Fig. 1b shows the ATP reduction improvement rate when treating human periodontal ligament fibroblasts with protamine sulfate after high-glucose treatment.
Fig. 1c shows the ATP reduction improvement rate when treating human periodontal ligament fibroblasts with methionine after high-glucose treatment.
Fig. 2a shows the oxygen consumption rate (OCR) reduction improvement rate when treating human periodontal ligament fibroblasts with calcium pantothenate after high-glucose treatment.
Fig. 2b shows the oxygen consumption rate (OCR) reduction improvement rate when treating human periodontal ligament fibroblasts with protamine sulfate after high-glucose treatment.
Fig. 2c shows the oxygen consumption rate (OCR) reduction improvement rate when treating human periodontal ligament fibroblasts with methionine after high-glucose treatment.
Fig. 3 shows OCR values (relative values) when treating human periodontal ligament fibroblasts with various test substances. The OCR values (relative values) are relative to the steady-state OCR value without treatment with any test substance, which is set to 1.
Fig. 4 shows the copy number of mitochondrial DNA (mtDNA) relative to nuclear DNA (nDNA) when treating human periodontal ligament fibroblasts with various test substances after high-glucose treatment.
Fig. 5a shows the ATP reduction improvement rate when treating human periodontal ligament fibroblasts with a combination of calcium pantothenate and protamine, methionine, rice bran, or ceramide after high-glucose treatment.
Fig. 5b shows the oxygen consumption rate (OCR) reduction improvement rate when treating human periodontal ligament fibroblasts with a combination of calcium pantothenate and protamine, methionine, rice bran, or ceramide after high-glucose treatment.
Fig. 6a shows the ATP reduction improvement rate when treating human periodontal ligament fibroblasts with a combination of panthenol and cinnamon oil or monosodium glutamate after high-glucose treatment.
Fig. 6b shows the oxygen consumption rate (OCR) reduction improvement rate when treating human periodontal ligament fibroblasts with a combination of panthenol and cinnamon oil or monosodium glutamate after high-glucose treatment.

Description of Embodiments

Embodiments encompassed by the present disclosure are described in more detail below. The present disclosure preferably encompasses, for example, a composition for activating mitochondria; however, the present disclosure is not limited to this, and encompasses everything disclosed herein and recognizable to those skilled in the art.
The composition for activating mitochondria encompassed in the present disclosure contains a specific component that can activate mitochondria. This composition may be referred to as "the composition of the present disclosure." Further, the specific component may be referred to as "the component of the present disclosure."
In an aspect, the composition of the present disclosure contains the specific components listed in the following table singly or in a combination of two or more.

Table 1b

| Chamomile extract |
|---|
| Cistanche extract |
| Olive leaf extract |
| Inositol |
| Eucommia extract |
| Arnica flower extract |

(continued)

| |
|---|
| Reduced palatinose |
| Honeysuckle leaf extract |
| Peony root extract |
| *Panax ginseng* root extract |
| Ginger extract |
| Placenta extract |
| *Lamium album* extract |
| Melilotus extract |
| Isoleucine |
| Willow extract |
| Wild grape extract |
| Valerian root extract |
| Fennel extract |
| Devil's claw extract |
| Hypericum extract |
| Cholecalciferol |
| Glycyrrhizic acid or a salt thereof |
| Senega extract |
| Allantoin |
| Rice bran extract |
| Fermented black garlic extract |
| Alanine |
| Purple brown rice extract |
| Protamine or a salt thereof |
| Wild thyme extract |
| Cinnamaldehyde |
| Turmeric rhizome extract |
| *Panax notoginseng* extract |
| Red ginger extract |
| Paramylon |
| Oat extract |
| Plantago herb extract |
| Cyclodextrin |
| *Houttuynia cordata* leaf extract |
| Arginine |
| Goji berry extract |
| Ceramide |
| Methionine |
| Rosemary leaf extract |
| Echinacea extract |

(continued)

| Yacon leaf extract |
| --- |
| Pantothenic acid |
| Panthenol |
| Soybean extract |
| Glutamic acid or a salt thereof |
| Horse chestnut seed extract |
| *Ginkgo biloba* extract |
| Reishi extract |
| Maca extract |
| Glycerin extract |
| Nettle root extract |
| Sweet tea extract |
| Pomegranate extract |

These components can also be purchased commercially. The extracts are preferably extracts with water, alcohol, or hydroalcohol. The alcohol is preferably ethanol and/or butylene glycol. The extracts may be dry substances or liquids.

These components can be broadly divided into compounds and extracts from organisms (e.g., whole or parts of animals or plants).

Examples of compounds include inositol, reduced palatinose, isoleucine, cholecalciferol, glycyrrhizic acid or a salt thereof, allantoin, alanine, protamine or a salt thereof, cinnamaldehyde, paramylon, cyclodextrin (particularly $\gamma$-cyclodextrin), arginine, ceramide, methionine, glutamic acid or a salt thereof, and the like. The salt of glycyrrhizic acid is preferably an alkali metal salt, and more preferably a sodium salt or a potassium salt. More specifically, dipotassium glycyrrhizinate and trisodium glycyrrhizinate are preferred. The salt of protamine is preferably hydrochloride or sulfate, and more preferably sulfate. The salt of glutamic acid is preferably an alkali metal salt, and more preferably a sodium salt or a potassium salt.

As the extracts of organisms, it is preferable to use extracts of parts that are generally used as topical or edible extracts.

Chamomile extract is not particularly limited as long as it is an extract of chamomile. Usable examples include extracts obtained from the stems, leaves, or flowers, among which extracted oils (e.g., essential oils) can be particularly preferably used.

Cistanche extract is preferably an extract of the fleshy stem of Cistanche. Cistanche extract is a parasitic plant of the Orobanchaceae family, and its extract is used as a natural medicine.

Olive leaf extract is an extract of olive leaves and is an ingredient used in supplements and topical preparations.

Eucommia extract is particularly preferably an extract of Eucommia leaves. Eucommia leaf extract is widely used as a supplement or the like.

Arnica flower extract is an extract obtained from the flowers (and roots) of *Arnica montana,* and is an ingredient used as a topical preparation or the like. Arnica is a plant of the Asteraceae family.

Honeysuckle leaf extract is an extract of honeysuckle leaves. Peony root extract is an extract of peony roots. *Panax ginseng* root extract is an extract of *Panax ginseng* root.

Ginger extract is preferably ginger rhizome extract. It can be used in topical preparations and foods with the expectation of functions, such as promoting blood circulation.

Placenta extract is an extract obtained from the placenta of mammals (particularly cows, pigs, and horses), and is used in cosmetics and the like.

*Lamium album* extract is an extract of the whole plant and/or flowers of *Lamium album,* which is a plant of the Lamiaceae family, and this extract is used in cosmetics and the like.

Melilotus extract is an extract of the whole plant of *Melilotus officinalis* (also known as sweet clover), which is a plant of the legume family, and this extract is used in foods and the like.

Willow extract is an extract of the bark and/or shoots of Western willow, and is used in supplements and the like.

Wild grape extract is an extract of wild grapes (mainly fruit (particularly seeds), stems, and leaves), and is used in foods and the like.

Valerian root extract is an extract of the root of *Valeriana officinalis* L., and is used in supplements and the like.

Fennel extract is an extract of fennel fruit, which is an umbelliferous plant, and this extract is used in cosmetics and the like.

Devil's claw extract is an extract of Devil's claw root (tuberous root), and is used in supplements and the like.

Hypericum extract is an extract of the flowers, stems, or leaves of hypericum, and is used in topical preparations and the like.

Senega extract is an extract of senega root and is used in cough suppressants/expectorants and the like.

Rice bran extract is an extract of rice bran.

Fermented black garlic extract is an extract of fermented black garlic obtained by fermenting garlic (bulb) for a certain period of time, and is used in supplements and the like.

Purple brown rice extract is an extract of brown rice with a reddish-purple color, such as ancient red rice or black rice, and is used in supplements and the like.

Wild thyme extract is an extract of the whole plant (particularly leaves and stems) of wild thyme, and is used in topical preparations and the like.

Turmeric rhizome extract is an extract of turmeric rhizome.

*Panax notoginseng* extract is an extract of *Panax notoginseng* (root) and is used in supplements and the like.

Although not particularly limited, in another aspect, particularly preferred among these components are the components listed in the following table.

Table 2

| Chamomile extract |
| --- |
| Cistanche extract |
| Olive leaf extract |
| Inositol |
| Eucommia extract |
| Arnica flower extract |
| Reduced palatinose |
| Honeysuckle leaf extract |
| Peony root extract |
| *Panax ginseng* root extract |
| Ginger extract |
| Placenta extract |
| *Lamium album* extract |
| Melilotus extract |
| Isoleucine |
| Willow extract |
| Wild grape extract |
| Valerian root extract |
| Fennel extract |
| Devil's claw extract |
| Hypericum extract |
| Cholecalciferol |
| Dipotassium glycyrrhizinate |
| Senega extract |
| Allantoin |
| Rice bran extract |
| Fermented black garlic extract |
| Alanine |

(continued)

| Purple brown rice extract |
| --- |
| Protamine sulfate |
| Wild thyme extract |
| Cinnamaldehyde |
| Turmeric rhizome extract |
| *Panax notoginseng* extract |

The content of the component of the present disclosure in the composition of the present disclosure in this aspect is not particularly limited as long as the effect is not impaired. For example, the content of the component is about 0.01 to 99.99 mass%.

In another aspect, the composition of the present disclosure comprises, as the component of the present disclosure, panthenol, pantothenic acid or a salt thereof, protamine or a salt thereof, and methionine or a salt thereof, singly or in a combination of two or more.

The salt of pantothenic acid is preferably an alkali metal salt or an alkaline earth metal salt. More specifically, the salt is more preferably a lithium salt, a sodium salt, a potassium salt, a calcium salt, a barium salt, or a magnesium salt; even more preferably a sodium salt or a calcium salt; and particularly preferably a calcium salt. These salts can be used singly or in a combination of two or more.

The salt of protamine is preferably an alkali metal salt or an alkaline earth metal salt. More specifically, the salt is more preferably a lithium salt, a sodium salt, a potassium salt, a calcium salt, a barium salt, or a magnesium salt; even more preferably a sodium salt or a calcium salt; and particularly preferably a calcium salt. The salt of protamine is also preferably sulfate or hydrochloride, and particularly preferably sulfate. These salts can be used singly or in a combination of two or more.

The salt of methionine is preferably an alkali metal salt or an alkaline earth metal salt. More specifically, the salt is more preferably a lithium salt, a sodium salt, a potassium salt, a calcium salt, a barium salt, or a magnesium salt; even more preferably a sodium salt or a calcium salt; and particularly preferably a calcium salt. The salt of methionine is also preferably sulfate or hydrochloride. These salts can be used singly or in a combination of two or more.

The content of the component of the present disclosure in the composition of the present disclosure in this aspect is not particularly limited as long as the effect is not impaired. For example, the content of the component is about 0.01 to 99.99 mass%.

In another aspect, the composition of the present disclosure comprises, as the component of the present disclosure, (A) pantothenic acid or a salt thereof, and (B) at least one selected from the group consisting of methionine or a salt thereof, protamine or a salt thereof, ceramide, and rice bran. Pantothenic acid or a salt thereof may be referred to as "the component (A)," and at least one selected from the group consisting of methionine or a salt thereof, protamine or a salt thereof, ceramide, and rice bran may be referred to as "the component (B)."

The salts of pantothenic acid, protamine, and methionine are as described above.

The origin of ceramide is not particularly limited, and usable examples include ceramides extracted from animals and plants, ceramides produced using microorganisms, or ceramides produced by chemical methods. Preferred among these are ceramides extracted from plants, such as corn germ, wheat, rice, soybean, millet, and spinach, or from microorganisms, such as yeast.

Rice bran is not particularly limited, and any known rice bran can be used. Usable examples of known rice bran include the pericarp, seed coat, perisperm, starch layer, etc. of brown rice, which are by-produced when white rice is produced by polishing brown rice. Rice bran with an oil content of more than 2 mass% is preferred. Further, rice bran extract obtained by squeezing rice bran or extracting it with water or alcohol (e.g., ethanol) can also be preferably used as the rice bran in the composition of the present disclosure.

The content ratio of the components (A) and (B) is not particularly limited as long as the effect is not impaired. For example, the content of the component (B) per part by mass of the component (A) is preferably about 0.1 to 10 parts by mass. The upper or lower limit of the range (0.1 to 10) may be, for example, 0.2, 0.3, 0.4, 0.5, 0.6, 0.7, 0.8, 0.9, 1, 2, 3, 4, 5, 6, 7, 8, or 9. For example, the range is more preferably about 0.2 to 5 parts by mass or about 0.2 to 3 parts by mass.

In the composition of the present disclosure in this aspect, the content of the component (A) is not particularly limited as long as the effect is not impaired. For example, the content of the component (A) is 0.001 to 20 mass% based on the total amount of the composition. The upper or lower limit of the range may be, for example, 0.005, 0.01, 0.05, 0.1, 0.5, 1, 2, 3, 4, 5, 7.5, 10, or 15. For example, the range is preferably 0.01 to 5 mass%.

Further, in the composition of the present disclosure in this aspect, the content of the component (B) is not particularly limited as long as the effect is not impaired. For example, the content of the component (B) is 0.001 to 20 mass% based on the total amount of the composition. The upper or lower limit of the range may be, for example, 0.005, 0.01, 0.05, 0.1, 0.5, 1, 2, 3, 4, 5, 7.5, 10, or 15. For example, the range is preferably 0.01 to 5 mass%.

When the composition of the present disclosure in this aspect contains methionine or a salt thereof, the content thereof is preferably within the content of the component (B) described above, more preferably 0.001 to 0.5 mass%, and even more preferably 0.01 to 0.1 mass%, based on the total amount of the composition.

When the composition of the present disclosure in this aspect contains protamine or a salt thereof, the content thereof is preferably within the content of the component (B) described above, more preferably 0.001 to 0.5 mass%, and even more preferably 0.005 to 0.1 mass%, based on the total amount of the composition.

When the composition of the present disclosure in this aspect contains ceramide, the content thereof is preferably within the content of the component (B) described above, more preferably 0.001 to 0.5 mass%, and even more preferably 0.01 to 0.1 mass%, based on the total amount of the composition.

When the composition of the present disclosure in this aspect contains rice bran, the content thereof is preferably within the content of the component (B) described above, more preferably 0.001 to 0.5 mass%, and even more preferably 0.01 to 0.1 mass%, based on the total amount of the composition.

In another aspect, the composition of the present disclosure comprises, as the component of the present disclosure, (C) panthenol and (D) at least one selected from the group consisting of cinnamon oil, and glutamic acid or a salt thereof. This composition may be referred to as "the composition of the present disclosure." Further, panthenol may be referred to as "the component (C)," and at least one selected from the group consisting of cinnamon oil, and glutamic acid or a salt thereof may be referred to as "the component (D)."

The salt of glutamic acid is preferably an alkali metal salt or an alkaline earth metal salt. More specifically, the salt is more preferably a lithium salt, a sodium salt, a potassium salt, a calcium salt, a barium salt, or a magnesium salt; even more preferably a sodium salt or a potassium salt; and particularly preferably a sodium salt. The salt of glutamic acid is also preferably sulfate or hydrochloride, and particularly preferably hydrochloride. These salts can be used singly or in a combination of two or more.

The content ratio of the components (C) and (D) is not particularly limited as long as the effect is not impaired. For example, the amount of the component (D) per part by mass of the component (C) is preferably about 0.1 to 10 parts by mass. The upper or lower limit of the range (0.1 to 10) may be, for example, 0.2, 0.3, 0.4, 0.5, 0.6, 0.7, 0.8, 0.9, 1, 2, 3, 4, 5, 6, 7, 8, or 9. For example, the range is more preferably about 0.2 to 5 parts by mass or about 0.2 to 3 parts by mass.

The content of the component (C) in the composition of the present disclosure in this aspect is not particularly limited as long as the effect is not impaired. For example, the content of the component (C) is 0.1 to 0.5 mass% based on the total amount of the composition. The upper or lower limit of the range may be, for example, 0.15, 0.2, 0.25, 0.3, 0.35, 0.4, or 0.45. For example, the range is preferably 0.15 to 0.45 mass% or 0.2 to 0.4 mass%.

The content of the component (D) in the composition of the present disclosure in this aspect is not particularly limited as long as the effect is not impaired. For example, the content of the component (D) is 0.001 to 1.3 mass% based on the total amount of the composition. The upper or lower limit of the range may be, for example, 0.005, 0.01, 0.02, 0.05, 0.1, 0.2, 0.5, 0.7, 1, or 1.2. For example, the range is preferably 0.005 to 1 mass% or 0.01 to 0.7 mass%.

When the composition of the present disclosure in this aspect contains cinnamon oil, the content thereof is preferably within the content of the component (D) described above, more preferably 0.001 to 1 mass%, even more preferably 0.002 to 0.5 mass%, and still even more preferably 0.01 to 0.1 mass%, based on the total amount of the composition.

When the composition of the present disclosure in this aspect contains glutamic acid or a salt thereof, the content thereof is preferably within the content of the component (B) described above, more preferably 0.01 to 0.3 mass%, and even more preferably 0.02 to 0.2 mass%, based on the total amount of the composition.

The mode of administration of the composition of the present disclosure is not particularly limited, but is preferably oral ingestion or topical application to the oral mucosa. That is, the composition of the present disclosure is preferably an oral composition or a composition for oral cavity. Oral ingestion of the composition of the present disclosure can activate mitochondria in cells (particularly cells in the oral cavity) and improve metabolic functions related to mitochondria (particularly improve the ability to consume oxygen of mitochondria). The composition of the present disclosure is preferably, for example, an oral pharmaceutical composition, a food composition (including a beverage composition and a food additive composition), or a composition for oral cavity.

The composition of the present disclosure contains the above component, and may further contain other components. Such other components can be selected appropriately depending on the field in which the composition is used. For example, pharmaceutically or food-hygienically acceptable carriers can be used.

For use as a pharmaceutical composition, examples of other components include pharmaceutically acceptable bases, carriers, and/or additives (e.g., solvents, dispersants, emulsifiers, buffers, stabilizers, excipients, binders, disintegrants, and lubricants). The form of the pharmaceutical composition is also not particularly limited, and examples include tablets, pills, powders, liquids, suspensions, emulsions, granules, capsules, creams, and poultices.

For use as a food composition, examples of other components include food-hygienically acceptable bases, carriers, additives, and other components and materials that can be used as foods. The form of the food composition is also not particularly limited, and examples include processed foods, health foods (e.g., nutritional supplements, food with nutrient function claims, foods for the sick, foods for specified health uses, and products with functional claims), supplements, and foods for the sick (e.g., hospital food, food for sick people, or nursing care food). These can be prepared by conventional methods. In particular, when preparing food compositions as health foods (e.g., nutritional supplements, food with nutrient function claims, foods for the sick, foods for specified health uses, and products with functional claims) or supplements, to facilitate continuous intake, it is preferable to prepare the compositions in the form of, for example, granules, capsules, tablets (including chewable tablets), or beverages (e.g., powdered drinks, energy drinks, and smoothies), among which capsules, tablets, powdered drinks, energy drinks, jellies, and gummies are preferred in terms of ease of intake, but are not particularly limited to these. When used as a food additive composition among food compositions, examples of its form include liquid, powder, flakes, granules, and paste.

For use as a composition for oral cavity, for example, the composition can be made into a form (dosage form), such as an ointment, a paste, a dermatological paste, a gel, a liquid, a spray, a mouthwash, a liquid dentifrice, a toothpaste, or a gum.

Other components that can be used include those known to be incorporated into compositions for oral cavity.

For example, surfactants, such as nonionic surfactants, anionic surfactants, and ampholytic surfactants, may be added. Specific examples of nonionic surfactants include sugar fatty acid esters, such as sucrose fatty acid esters, maltose fatty acid esters, and lactose fatty acid esters; fatty acid alkanolamides; glycerin fatty acid esters; sorbitan fatty acid esters; fatty acid monoglyceride; polyoxyethylene alkyl ethers with a polyoxyethylene addition factor of 8 to 10, and 13 to 15 carbon atoms in the alkyl group; polyoxyethylene alkyl phenyl ethers with a polyoxyethylene addition factor of 10 to 18, and 9 carbon atoms in the alkyl group; diethyl sebacate; polyoxyethylene hydrogenated castor oil; and fatty acid polyoxyethylene sorbitan. Examples of anionic surfactants include sulfates, such as sodium lauryl sulfate and sodium polyoxyethylene lauryl ether sulfate; sulfosuccinates, such as sodium lauryl sulfosuccinate and sodium polyoxyethylene lauryl ether sulfosuccinate; acyl amino acid salts, such as sodium cocoyl sarcosine and sodium lauroyl methylalanine; and sodium cocoyl methyl taurine. Examples of ampholytic surfactants include betaine acetate activators, such as betaine lauryl dimethylamino acetate and coconut oil fatty acid amide propyldimethylamino acetate betaine; imidazoline activators, such as sodium N-cocoyl-N-carboxymethyl-N-hydroxyethylethylenediamine; and amino acid activators, such as N-lauryl diaminoethyl glycine. These surfactants can be added singly or in a combination of two or more. The amount of the surfactant added is typically 0.1 to 5 mass% based on the total amount of the composition.

Examples of flavoring agents that can be added include menthol, carboxylic acid, anethole, eugenol, methyl salicylate, limonene, ocimene, n-decyl alcohol, citronellal, $\alpha$-terpineol, methyl acetate, citronellyl acetate, methyleugenol, cineol, linalool, ethyl linalool, thymol, spearmint oil, peppermint oil, lemon oil, orange oil, sage oil, rosemary oil, cinnamon oil, beefsteak plant oil, wintergreen oil, clove oil, eucalyptus oil, pimento oil, d-camphor, d-borneol, fennel oil, cinnamon oil, mint oil, and vanillin. These flavoring agents can be used singly or in a combination of two or more, and the amount of the flavoring agents added may be, for example, 0.001 to 1.5 mass% based on the total amount of the composition.

Examples of sweeteners include saccharin sodium, acesulfame potassium, stevioside, neohesperidin dihydrochalcone, perillatin, thaumatin, aspartylphenylalanine methyl ester, and p-methoxycinnamic aldehyde. The amount of the sweeteners added may be, for example, 0.01 to 1 mass% based on the total amount of the composition.

Further, wetting agents such as sorbit, ethylene glycol, propylene glycol, glycerol, 1,3-butylene glycol, polypropylene glycol, xylitol, maltitol, lactitol, and polyoxyethylene glycol can be added singly or in a combination of two or more.

Preservatives such as the following can be added: parabens, such as methylparaben, ethylparaben, propylparaben, and butylparaben, sodium benzoate, phenoxyethanol, and alkyldiaminoethylglycine hydrochloride.

Colorants such as the following can be added: legally permitted pigments such as blue No. 1, yellow No. 4, red No. 202, and green No. 3; mineral-based pigments such as ultramarine, enhanced ultramarine, and ferric hexacyanoferrate; and titanium oxide.

pH Adjusters such as the following can be added: citric acid, phosphoric acid, malic acid, pyrophosphoric acid, lactic acid, tartaric acid, glycerophosphoric acid, acetic acid, nitric acid, chemically possible salts thereof, and sodium hydroxide. These pH adjusters can be added singly or in a combination of two or more such that the composition has a pH of 4 to 8, and preferably 5 to 7. The amount of the pH adjuster may be, for example, 0.01 to 2 wt%.

The following medicinal ingredients can be added singly or in a combination of two or more: vitamin E, such as dl-$\alpha$-tocopherol acetate, tocopherol succinate, or tocopherol nicotinate; ampholytic sterilizers, such as dodecyl diamino ethyl glycine; nonionic sterilizers, such as triclosan, isopropyl methylphenol, and hinokitiol; anionic sterilizers, such as sodium lauroyl sarcosine; cationic sterilizers, such as cetylpyridinium chloride, chlorhexidine hydrochloride, benzalkonium chloride, and benzethonium chloride; enzymes, such as dextranase, amylase, protease, mutanase, lysozyme, and lytic enzymes; alkali metal monofluorophosphates, such as sodium monofluorophosphate and potassium monofluorophosphate; fluorides, such as sodium fluoride and stannous fluoride; tranexamic acid, epsilon aminocaproic acid, aluminum chlorohydroxy allantoin, dihydrocholesterol, glycyrrhetinic acid, sodium copper chlorophyllin, glycerophosphate, chlorophyll, sodium chloride, caropeptide, carbazochrome, hinokitiol, potassium nitrate, and palatinit.

Bases such as the following can also be added: alcohols, silicon, apatite, white Vaseline, paraffin, liquid paraffin, microcrystalline wax, squalane, and Plastibase.

The composition of the present disclosure can be preferably used for activating mitochondria in cells. More specifically, the composition of the present disclosure can preferably improve, for example, the reduction in the oxygen consumption rate caused by high glucose or oxidative stress load. Therefore, the composition of the present disclosure can also be preferably used to improve metabolic abnormalities in human-derived cells of diabetic patients or those at risk of developing diabetes. For example, in diabetic conditions, the metabolic function of periodontal tissues is impaired, which may accelerate the progression of periodontal disease in diabetic patients. For this reason, in particular, the composition of the present disclosure can also be preferably used to improve metabolic abnormalities in periodontal tissue cells (e.g., periodontal ligament cells), and thus to inhibit and/or improve the progression of periodontal disease. In addition, it is possible to not only improve the mitochondrial metabolism of cells in the oral cavity (e.g., gingival cells and periodontal ligament cells), but also activate the metabolic functions of various cells or suppresses the decline of metabolic functions. For this reason, the composition of the present disclosure can be used not only to inhibit and/or improve the progression of periodontal disease, but also to improve the metabolic functions of other tissues that are impaired, for example, in diabetic or pre-diabetic conditions, or with aging. For example, the composition of the present disclosure can be used to inhibit and/or improve obesity, renal dysfunction, retinopathy, neuropathy, cardiovascular disease, brain dysfunction, dementia, frailty, muscle weakness, and osteoporosis.

The subject for ingestion or application of the composition of the present disclosure is not particularly limited. For example, the composition of the present disclosure can preferably be ingested or applied to healthy individuals. From the viewpoint that the effect can be preferably achieved, subjects with metabolic abnormalities (particularly subjects with impaired mitochondrial metabolism due to, for example, increased intracellular oxidative stress) are preferred, and more specifically, subjects with metabolic abnormalities caused by, for example, high-glucose loads are more preferred. Further, as described above, by improving the mitochondrial metabolism of cells in the oral cavity (e.g., gingival cells and periodontal ligament cells), it is possible to activate the metabolic functions of cells in the oral cavity or suppress the decline of metabolic functions, which is expected to help maintain oral health (e.g., prevent periodontal disease). Therefore, subjects with gingival inflammation or periodontal disease are also preferred.

Although the time of ingestion or application is not particularly limited, for example, the composition of the present disclosure may be used to be ingested or applied before or after a meal (e.g., within 0.5 or 1 hour before or after a meal). It is more preferable that the meal contains carbohydrates (particularly carbohydrates that can be converted into glucose when absorbed into the body) and lipids. The composition of the present disclosure can also be preferably used to prevent recurrence during or after treatment of periodontal disease, for example.

Although not particularly limited, cells in which metabolic abnormalities can be suppressed by ingestion or application of the composition of the present disclosure are preferably cells in the oral cavity, among which gingival cells and periodontal ligament cells (particularly gingival fibroblasts and periodontal ligament fibroblasts) are preferred. Periodontal ligament cells are most preferred because they are known to be strongly involved in immune responses, inflammatory reactions, and alveolar bone resorption in periodontal disease.

In the present specification, the terms "comprising" and "containing" also include consisting essentially of and consisting of. The present disclosure encompasses any combination of the elements described in the present specification.

The various characteristics (e.g., properties, structures, functions) described in each embodiment of the present disclosure can be combined in any way in specifying the subject matter encompassed in the present disclosure. Specifically, the present disclosure encompasses all subject matter formed by any possible combination of the characteristics described in the present specification.

Examples

The embodiments of the present disclosure are described with reference to examples in more detail below. However, the embodiments of the present disclosure are not limited to the following examples.

(1) Examination of Various Components

After human periodontal ligament fibroblasts (HPDLFs) were treated with test samples, the oxygen consumption rate (OCR) in the cells were measured. The more specific procedures were as follows.

Measurement of Oxygen Consumption Rate (OCR) in Periodontal Ligament Fibroblasts

The OCR was measured using an Extracellular OCR Plate Assay Kit (E297, DOJINDO). Human periodontal ligament fibroblasts (HPDLFs) were seeded in a 96-well plate at a density of $2 \times 10^5$ cells/100 $\mu$L/well and cultured overnight. Thereafter, the cells were treated with each test substance (sample; final concentration: 1 $\mu$g/mL) for 24 hours.

The OCR was measured as follows. 100 $\mu$L of an oxygen probe reaction solution, which has the property of increasing phosphorescence intensity when the oxygen concentration in the culture medium decreases, was added to each well. A drop of mineral oil was then added to each well to prevent oxygen from entering from the air. The phosphorescence intensity was measured using a Cytation 5 plate reader (BioTek Instruments), and the OCR in the cells was calculated using the Stern-Volmer equation. The OCR value (relative value) of each test substance is shown as a value relative to the steady-state OCR value without treatment with any test substance, which is set to 1. That is, since the steady-state OCR value without treatment with any test substance is set to 1, if the OCR value doubles after treatment with the test substance, the OCR value is 2.

Table 3 shows the type and amount of each sample, as well as the results obtained.

Table 3

| | OCR value |
|---|---|
| Test substance (sample) | Relative value |
| Chamomile extract | 11.0 |
| Cistanche extract | 10.1 |
| Olive leaf extract | 7.8 |
| Inositol | 7.4 |
| Eucommia extract | 7.0 |
| Arnica flower extract | 6.7 |
| Reduced palatinose | 6.7 |
| Honeysuckle leaf extract | 6.6 |
| Peony root extract | 6.6 |
| *Panax ginseng* root extract | 6.6 |
| Ginger extract | 6.5 |
| Placenta extract | 6.4 |
| *Lamium album* extract | 6.3 |
| Melilotus extract | 6.1 |
| Isoleucine | 6.1 |
| Willow extract | 5.8 |
| Wild grape extract | 5.5 |
| Valerian root extract | 5.0 |
| Fennel extract | 4.7 |
| Devil's claw extract | 4.6 |
| Hypericum extract | 4.6 |
| Cholecalciferol | 4.3 |
| Dipotassium glycyrrhizinate | 4.2 |
| Senega extract | 4.1 |
| Allantoin | 4.0 |
| Rice bran extract | 3.9 |
| Fermented black garlic extract | 3.8 |
| Alanine | 3.6 |
| Purple brown rice extract | 3.3 |
| Protamine sulfate | 3.3 |

(continued)

|  | OCR value |
| Test substance (sample) | Relative value |
| Wild thyme extract | 3.2 |
| Cinnamaldehyde | 3.2 |
| Turmeric rhizome extract | 3.2 |
| *Panax notoginseng* extract | 3.0 |
| Red ginger extract | 2.8 |
| Paramylon | 2.7 |
| Oat extract | 2.5 |
| Plantago herb extract | 2.4 |
| $\gamma$-Cyclodextrin | 2.4 |
| *Houttuynia cordata* leaf extract | 2.3 |
| L-arginine | 2.3 |
| Goji berry extract | 2.3 |
| Ceramide | 2.2 |
| L-methionine | 2.0 |
| Rosemary leaf extract | 1.9 |
| Echinacea extract | 1.9 |
| Yacon leaf extract | 1.9 |
| Pantothenic acid | 1.8 |
| Panthenol | 1.6 |
| Trisodium glycyrrhizinate | 1.6 |
| Soybean extract | 1.5 |
| Monosodium L-glutamate | 1.5 |
| Horse chestnut seed extract | 1.4 |
| *Ginkgo biloba* extract | 1.4 |
| Reishi extract | 1.2 |
| Maca extract | 1.2 |
| Glycerin extract | 1.2 |
| Nettle root extract | 1.2 |
| Sweet tea extract | 1.1 |
| Pomegranate extract | 1.1 |

(2) Examination of Specific Components

After human periodontal ligament fibroblasts (HPDLFs) were treated with test samples, the intracellular ATP concentration and the oxygen consumption rate (OCR) in the cells were measured. The more specific procedures were as follows.

The test substances (samples) used were calcium pantothenate, protamine sulfate, and methionine. All test substances were dissolved in the medium used in the test for use as solutions.

Measurement of Intracellular ATP in Periodontal Ligament

Fibroblasts

Intracellular ATP was measured using a Luminescent ATP Detection Assay Kit (ab113849; Abcam). Human periodontal ligament fibroblasts (HPDLFs) were seeded in a 48-well plate at a density of $0.12 \times 10^5$ cells/100 μL/well and cultured for 48 hours. Thereafter, the cells were pre-treated with each test substance (sample) for 24 hours and subsequently cultured for an additional 72 hours in the presence of 50 mM D-glucose (Sigma Aldrich). Controls were treated in the same manner with 50 mM L-glucose. Then, 50 μL of cell lysate was added for 5 minutes to lyse the cells and stabilize ATP. Further, D-luciferase reagent was added, followed by incubation for 10 minutes in the dark. Chemiluminescence from the luciferase reaction was measured using a Cytation 5 plate reader (BioTek Instruments), and the ATP concentration (μM) was measured using a standard curve. The ATP reduction improvement rate (%) of each sample was calculated using the following formula.

$$\text{ATP reduction improvement rate (\%)} = \frac{sample - D\text{-}glucose}{L\text{-}glucose - D\text{-}glucose} \times 100$$

That is, the ATP reduction improvement rate (%) represents the percentage (%) obtained by dividing a value obtained by subtracting an ATP value measured after treatment with only D-glucose from an ATP value measured after treatment with a sample and then D-glucose, by a value obtained by subtracting an ATP value measured after treatment with only D-glucose from an ATP value measured after treatment with only L-glucose.

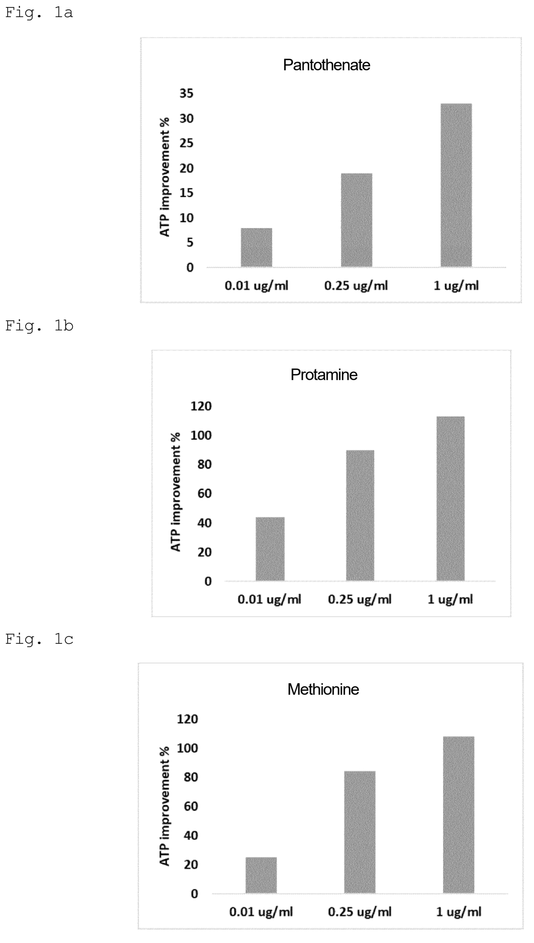

The results are shown in Figs. 1a to 1c. In the figures, "Pantothenate" represents calcium pantothenate, and "Protamine" represents protamine sulfate. When concentrations are indicated in the figures, these concentrations represent the concentrations of each test substance in the culture medium. The same applies to the following figures.

Measurement of Oxygen Consumption Rate (OCR) in Periodontal Ligament Fibroblasts

The OCR was measured using an Extracellular OCR Plate Assay Kit (E297, DOJINDO). Human periodontal ligament fibroblasts (HPDLFs) were seeded in a 96-well plate at a density of $2 \times 10^5$ cells/100 μL/well and cultured overnight. Thereafter, the cells were pre-treated with each test substance (sample) for 24 hours and subsequently cultured for an additional 24 hours in the presence of 100 mM D-glucose (Sigma Aldrich). Controls were treated in the same manner with 100 mM L-glucose. 100 μL of an oxygen probe reaction solution, which has the property of increasing phosphorescence intensity when the oxygen concentration in the culture medium decreases, was added to each well. A drop of mineral oil was then added to each well to prevent oxygen from entering from the air. The phosphorescence intensity was measured using a Cytation 5 plate reader (BioTek Instruments), and the OCR in the cells was calculated using the Stern-Volmer equation. The OCR improvement rate (%) of each sample was calculated using the following formula.

$$\text{OCR reduction improvement rate (\%)} = \frac{sample - D\text{-}glucose}{L\text{-}glucose - D\text{-}glucose} \times 100$$

That is, the OCR reduction improvement rate (%) represents the percentage (%) obtained by dividing a value obtained by subtracting an OCR value measured after treatment with only D-glucose from an OCR value measured after treatment with a sample and then D-glucose, by a value obtained by subtracting an OCR value measured after treatment with only D-glucose from an OCR value measured after treatment with only L-glucose.

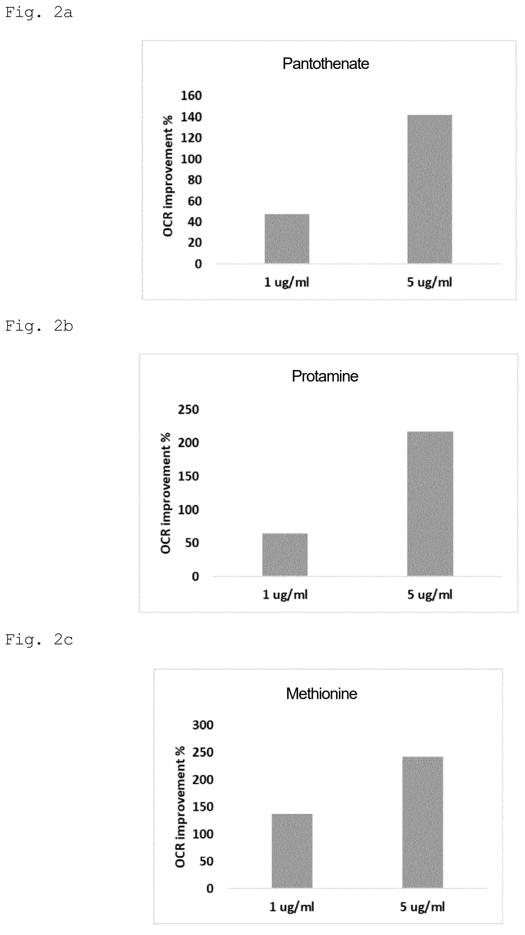

The results are shown in Figs. 2a to 2c.

Measurement of Oxygen Consumption Rate (OCR) in Periodontal Ligament Fibroblasts (No High-Glucose Load)

The OCR was measured using an Extracellular OCR Plate Assay Kit (E297, DOJINDO). Human periodontal ligament fibroblasts (HPDLFs) were seeded in a 96-well plate at a density of $2 \times 10^5$ cells/100 μL/well and cultured overnight. Thereafter, the cells were treated with each test substance (sample; final concentration: 1 μg/mL) for 24 hours.

The OCR was measured as follows. 100 μL of an oxygen probe reaction solution, which has the property of increasing phosphorescence intensity when the oxygen concentration in the culture medium decreases, was added to each well. A drop of mineral oil was then added to each well to prevent oxygen from entering from the air. The phosphorescence intensity was measured using a Cytation 5 plate reader (BioTek Instruments), and the OCR in the cells was calculated using the Stern-Volmer equation. The OCR value (relative value) of each test substance is shown as a value relative to the steady-state OCR value without treatment with any test substance, which is set to 1. That is, since the steady-state OCR value without treatment with any test substance is set to 1, if the OCR value doubles after treatment with the test substance, the OCR value is 2.

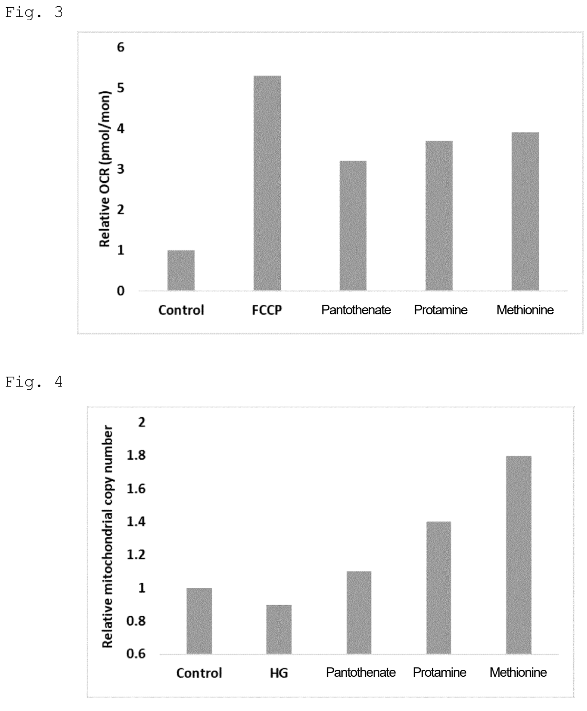

The results are shown in Fig. 3. As a positive control substance, the uncoupler FCCP was used at a final concentration of 2 μM. FCCP is carbonyl cyanide-p-trifluoromethoxyphenylhydrazone. Only the medium was used as a control.

Method for Measuring Mitochondria DNA in Periodontal Ligament Fibroblasts

Human periodontal ligament fibroblasts (HPDLFs) were seeded in a 12-well plate at a density of $0.9 \times 10^5$ cells/well. Two days later, the cells were treated with each test substance (sample; final concentration: 1 μg/mL) for 24 hours and then cultured for 3 days in the presence of 50 mM D-glucose (Sigma Aldrich, USA). Controls were treated in the same manner with 50 mM L-glucose. DNA was isolated using a NucleoSpin Tissue Extraction Kit (Takara, Japan). Quantitative PCR was performed on the isolated DNA using TB Green Fast qPCR Mix (Takara, Japan) and ABI 7500 fast (Thermo Fisher Scientific), and the copy number of mitochondrial DNA (mtDNA) was calculated relative to that of nuclear DNA (nDNA).

The results are shown in Fig. 4. In the figure, "HG" indicates that only 50 mM D-glucose was applied (no test substance was applied). Further, "Control" indicates that only 50 mM L-glucose was applied (no test substance was applied).

An increase in the relative copy number of mtDNA to nDNA is considered to mean an increase in mitochondria and, in turn, an improvement in metabolic functions related to mitochondria.

(3) Examination 1 of Combinations of Specific Components

After human periodontal ligament fibroblasts (HPDLFs) were treated with test samples and high glucose, the intracellular ATP concentration and the oxygen consumption rate (OCR) in the cells were measured. The more specific procedures were as follows.

Measurement of Intracellular ATP in Periodontal Ligament Fibroblasts

Intracellular ATP was measured using a Luminescent ATP Detection Assay Kit (ab113849; Abcam). Human periodontal ligament fibroblasts (HPDLFs) were seeded in a 48-well plate at a density of $0.12 \times 10^5$ cells/100 μL/well and cultured for 48 hours. Thereafter, the cells were pre-treated with each test substance (sample) for 24 hours and subsequently cultured for an additional 72 hours in the presence of 50 mM D-glucose (Sigma Aldrich). Controls were treated in the same manner with 50 mM L-glucose. Then, 50 μL of cell lysate was added for 5 minutes to lyse the cells and stabilize ATP. Further, D-luciferase reagent was added, followed by incubation for 10 minutes in the dark. Chemiluminescence from the luciferase reaction was measured using a Cytation 5 plate reader (BioTek Instruments), and the ATP concentration (μM) was measured using a standard curve. The ATP reduction improvement rate (%) of each sample was calculated using the following formula.

$$\text{ATP reduction improvement rate (\%)} = \frac{sample - D\text{-}glucose}{L\text{-}glucose - D\text{-}glucose} \times 100$$

That is, the ATP reduction improvement rate (%) represents the percentage (%) obtained by dividing a value obtained by subtracting an ATP value measured after treatment with only D-glucose from an ATP value measured after treatment with a sample and then D-glucose, by a value obtained by subtracting an ATP value measured after treatment with only D-glucose from an ATP value measured after treatment with only L-glucose.

Measurement of Oxygen Consumption Rate (OCR) in Periodontal Ligament Fibroblasts

The OCR was measured using an Extracellular OCR Plate Assay Kit (E297, DOJINDO). Human periodontal ligament fibroblasts (HPDLFs) were seeded in a 96-well plate at a density of $2 \times 10^5$ cells/100 μL/well and cultured overnight. Thereafter, the cells were pre-treated with each test substance (sample) for 24 hours and subsequently cultured for an additional 24 hours in the presence of 100 mM D-glucose (Sigma Aldrich). Controls were treated in the same manner with 100 mM L-glucose. 100 μL of an oxygen probe reaction solution, which has the property of increasing phosphorescence intensity when the oxygen concentration in the culture medium decreases, was added to each well. A drop of mineral oil was then added to each well to prevent oxygen from entering from the air. The phosphorescence intensity was measured using a Cytation 5 plate reader (BioTek Instruments), and the OCR in the cells was calculated using the Stern-Volmer equation. The OCR improvement rate (%) of each sample was calculated using the following formula.

$$\text{OCR reduction improvement rate (\%)} = \frac{sample - D\text{-}glucose}{L\text{-}glucose - D\text{-}glucose} \times 100$$

That is, the OCR reduction improvement rate (%) represents the percentage (%) obtained by dividing a value obtained by subtracting an OCR value measured after treatment with only D-glucose from an OCR value measured after treatment with a sample and then D-glucose, by a value obtained by subtracting an OCR value measured after treatment with only D-glucose from an OCR value measured after treatment with only L-glucose.

Tables 4a and 4b show the type and amount of each sample, as well as the results obtained. All samples were used as aqueous solutions. In these tables, "Pantothenate" represents calcium pantothenate. Further, "ALA" represents 5-aminolevulinic acid. Commercially available rice-derived ceramide was used as "Ceramide." Further, commercially available rice bran water extract was used as "Rice Bran."

The concentration of each sample aqueous solution is expressed as % (w/v), but since this value is almost the same as the % (w/w) value (with % (w/w) being only slightly larger), it is acceptable to interpret it as % (w/w) (i.e., mass%).

Table 4a

| Test substance (sample) | Amount | ATP reduction improvement rate (%) | OCR reduction improvement rate (%) |
|---|---|---|---|
| Pantothenate | 1 ug/ml | 33 | 48 |
| Protamine sulfate | 250 ng/ml | 100 | 64 |
| Pantothenate + protamine sulfate | 1 ug/ml + 250 ng/ml | 200 | 178 |
| L-Methionine | 1 ug/ml | 86 | 137 |
| Pantothenate + L-methionine | 1 ug/ml + 1 ug/ml | 153 | 316 |
| Rice bran | 1 ug/ml | 97 | 95 |
| Pantothenate + rice bran | 1 ug/ml + 1 ug/ml | 136 | 133 |
| Ceramide | 1 ug/ml | 92 | 40 |
| Pantothenate + ceramide | 1 ug/ml + 1 ug/ml | 134 | 113 |

Table 4b

| Test substance (sample) | Amount | ATP reduction improvement rate (%) |
|---|---|---|
| Protamine | 1 ug/ml | 33 |
| 5ALA | 1 ug/ml | 67 |
| Protamine + 5ALA | 1 ug/ml + 1 ug/ml | 33 |
| Yerba mate extract | 1 ug/ml | 16 |
| Protamine + yerba mate extract | 1 ug/ml + 1 ug/ml | 16 |
| Placenta extract | 1 ug/ml | 5 |
| Protamine + placenta extract | 1 ug/ml + 1 ug/ml | 32 |
| Protamine + horse chestnut seed extract | 1 ug/ml | 46 |
| Protamine + sweet tea extract | 1 ug/ml + 1 ug/ml | 21 |

As shown in Table 4a, it was found that when calcium pantothenate was used in combination with protamine, methionine, rice bran, or ceramide, both the ATP reduction improvement rate and the OCR reduction improvement rate showed excellent values.

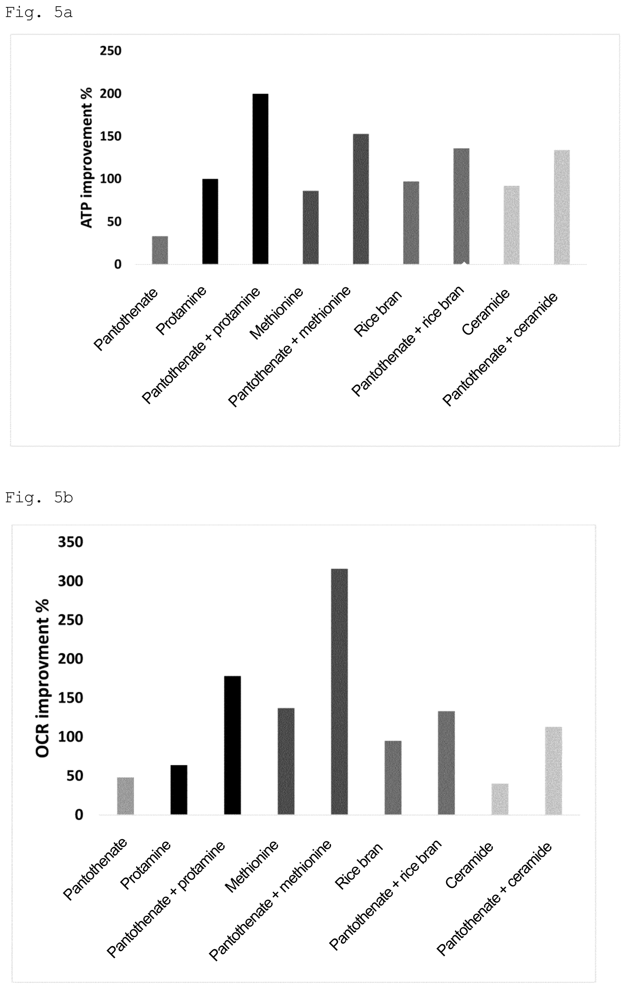

The results shown in Table 4a are also shown as graphs in figures. Specifically, the results for the ATP reduction improvement rate in Table 4a are shown in Fig. 5a, and the results for the OCR reduction improvement rate in Table 4b are shown in Fig. 5b.

(4) Examination 2 of Combinations of Specific Components

After human periodontal ligament fibroblasts (HPDLFs) were treated with test samples and high glucose, the intracellular ATP concentration and the oxygen consumption rate (OCR) in the cells were measured. The more specific procedures were as follows.

Measurement of Intracellular ATP in Periodontal Ligament Fibroblasts

Intracellular ATP was measured using a Luminescent ATP Detection Assay Kit (ab113849; Abcam). Human periodontal ligament fibroblasts (HPDLFs) were seeded in a 48-well plate at a density of $0.12 \times 10^5$ cells/100 $\mu$L/well and cultured for 48 hours. Thereafter, the cells were pre-treated with each test substance (sample) for 24 hours and subsequently cultured for an additional 72 hours in the presence of 50 mM D-glucose (Sigma Aldrich). Controls were treated in the same manner with 50 mM L-glucose. Then, 50 $\mu$L of cell lysate was added for 5 minutes to lyse the cells and stabilize ATP. Further, D-luciferase reagent was added, followed by incubation for 10 minutes in the dark. Chemiluminescence from the luciferase reaction was measured using a Cytation 5 plate reader (BioTek Instruments), and the ATP concentration ($\mu$M) was measured using a standard curve. The ATP reduction improvement rate (%) of each sample was calculated using the following formula.

$$\text{ATP reduction improvement rate (\%)} = \frac{sample - D\text{-}glucose}{L\text{-}glucose - D\text{-}glucose} \times 100$$

That is, the ATP reduction improvement rate (%) represents the percentage (%) obtained by dividing a value obtained by subtracting an ATP value measured after treatment with only D-glucose from an ATP value measured after treatment with a sample and then D-glucose, by a value obtained by subtracting an ATP value measured after treatment with only D-glucose from an ATP value measured after treatment with only L-glucose.

Measurement of Oxygen Consumption Rate (OCR) in Periodontal Ligament Fibroblasts

The OCR was measured using an Extracellular OCR Plate Assay Kit (E297, DOJINDO). Human periodontal ligament fibroblasts (HPDLFs) were seeded in a 96-well plate at a density of $2 \times 10^5$ cells/100 $\mu$L/well and cultured overnight. Thereafter, the cells were pre-treated with each test substance (sample) for 24 hours and subsequently cultured for an additional 24 hours in the presence of 100 mM D-glucose (Sigma Aldrich). Controls were treated in the same manner with 100 mM L-glucose. 100 $\mu$L of an oxygen probe reaction solution, which has the property of increasing phosphorescence intensity when the oxygen concentration in the culture medium decreases, was added to each well. A drop of mineral oil was then added to each well to prevent oxygen from entering from the air. The phosphorescence intensity was measured using a Cytation 5 plate reader (BioTek Instruments), and the OCR in the cells was calculated using the Stern-Volmer equation. The OCR improvement rate (%) of each sample was calculated using the following formula.

$$\text{OCR reduction improvement rate (\%)} = \frac{sample - D\text{-}glucose}{L\text{-}glucose - D\text{-}glucose} \times 100$$

That is, the OCR reduction improvement rate (%) represents the percentage (%) obtained by dividing a value obtained by subtracting an OCR value measured after treatment with only D-glucose from an OCR value measured after treatment with a sample and then D-glucose, by a value obtained by subtracting an OCR value measured after treatment with only D-glucose from an OCR value measured after treatment with only L-glucose.

Table 5 shows the type and amount of each sample, as well as the results obtained. All samples were used as aqueous solutions. "Cinnamon oil" represents cinnamon oil that complies with the Japanese Pharmacopoeia standards.

The concentration of each sample aqueous solution is expressed as % (w/v), but since this value is almost the same as the % (w/w) value (with % (w/w) being only slightly larger), it is acceptable to interpret it as % (w/w) (i.e., mass%).

Table 5

| Test substance (sample) | Amount | ATP reduction improvement rate (%) | OCR reduction improvement rate (%) |
|---|---|---|---|
| Panthenol | 1 ug/ml | 37 | 81 |
| Cinnamon oil | 1 ug/ml | 59 | 21 |

(continued)

| Test substance (sample) | Amount | ATP reduction improvement rate (%) | OCR reduction improvement rate (%) |
|---|---|---|---|
| Panthenol + cinnamon oil | 1 ug/ml + 1 ug/ml | 115 | 198 |
| Monosodium L-glutamate | 1 ug/ml | 57 | 31 |
| Panthenol + monosodium L-glutamate | 1 ug/ml + 1 ug/ml | 133 | 158 |

As shown in Table 5, it was found that when panthenol was used in combination with cinnamon oil, or glutamic acid or a salt thereof, both the ATP reduction improvement rate and the OCR reduction improvement rate showed excellent values.

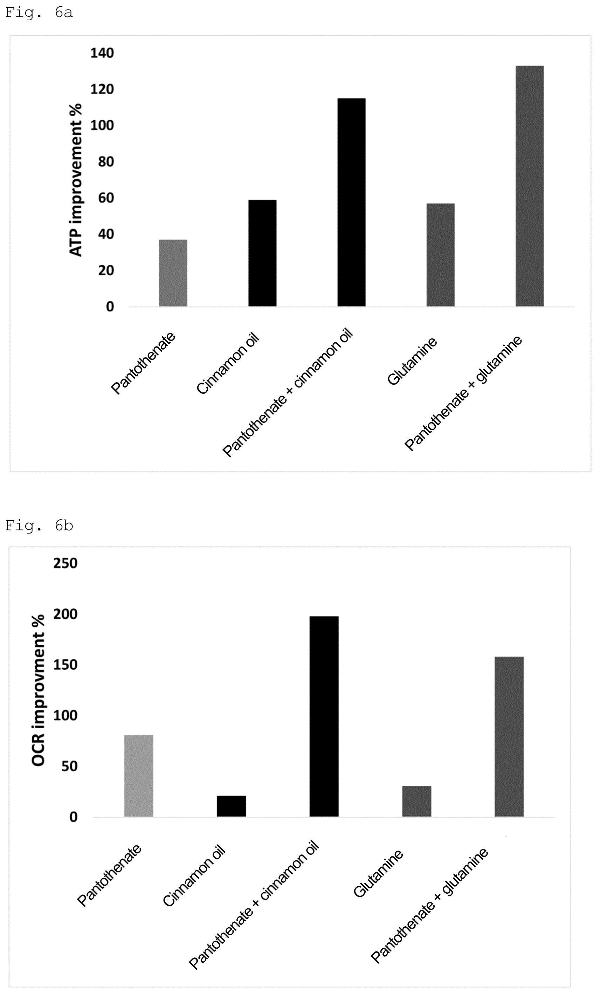

The results shown in Table 5 are also shown as graphs in figures. Specifically, the results for the ATP reduction improvement rate in Table 5 are shown in Fig. 6a, and the results for the OCR reduction improvement rate in Table 5 are shown in Fig. 6b. In these figures, "Glutamine" represents "monosodium L-glutamate."

**Claims**

1.   A compound for use in improving periodontal disease, the compound being at least one compound selected from the group consisting of:

panthenol,
pantothenic acid or a salt thereof,
protamine or a salt thereof, and
methionine or a salt thereof.

2.   The compound for use in improving periodontal disease according to claim 1, wherein the compound comprises:

(A) pantothenic acid or a salt thereof, and
(B) at least one selected from the group consisting of methionine or a salt thereof, protamine or a salt thereof, ceramide, and rice bran.

3.   The compound for use in improving periodontal disease according to claim 1, wherein the compound comprises:

(C) panthenol, and
(D) at least one selected from the group consisting of cinnamon oil, and glutamic acid or a salt thereof.

4.   The compound for use in improving periodontal disease according to any one of claims 1 to 3, wherein the periodontal disease is periodontal disease in a diabetic patient.

5.   A compound for use in activating mitochondria, the compound being at least one compound selected from the group consisting of:

panthenol,
pantothenic acid or a salt thereof,
protamine or a salt thereof, and
methionine or a salt thereof.

6.   The compound for use in activating mitochondria according to claim 5, wherein the compound comprises:

(A) pantothenic acid or a salt thereof, and
(B) at least one selected from the group consisting of methionine or a salt thereof, protamine or a salt thereof, ceramide, and rice bran.

7.   The compound for use in activating mitochondria according to claim 5, wherein the compound comprises:

(C) panthenol, and

(D) at least one selected from the group consisting of cinnamon oil, and glutamic acid or a salt thereof.

8. The compound for use in activating mitochondria according to any one of claims 5 to 7, wherein the mitochondria are mitochondria in periodontal ligament cells.

9. A compound for use in improving a symptom caused by diabetes, prediabetes, or an age-related metabolic disorder, the compound being at least one compound selected from the group consisting of:

panthenol,
pantothenic acid or a salt thereof,
protamine or a salt thereof, and
methionine or a salt thereof.

10. The compound for use in improving a symptom caused by diabetes, prediabetes, or an age-related metabolic disorder according to claim 9, wherein the compound comprises:

(A) pantothenic acid or a salt thereof, and
(B) at least one selected from the group consisting of methionine or a salt thereof, protamine or a salt thereof, ceramide, and rice bran.

11. The compound for use in improving a symptom caused by diabetes, prediabetes, or an age-related metabolic disorder according to claim 9, wherein the compound comprises:

(C) panthenol, and
(D) at least one selected from the group consisting of cinnamon oil, and glutamic acid or a salt thereof.

12. The compound for use in improving a symptom caused by diabetes, prediabetes, or an age-related metabolic disorder according to any one of claims 9 to 11, wherein the symptom caused by diabetes, prediabetes, or an age-related metabolic disorder is obesity, renal dysfunction, retinopathy, neuropathy, cardiovascular disease, brain dysfunction, dementia, frailty, muscle weakness, or osteoporosis.

13. An oral composition or composition for oral cavity, comprising the compound according to any one of claims 1 to 12.

14. A food composition, comprising the compound according to any one of claims 1 to 12.

15. The composition for oral cavity according to claim 13, which is an ointment, a paste, a dermatological paste, a gel, a liquid, a spray, a mouthwash, a liquid dentifrice, a toothpaste, or a gum.

Fig. 1a

Fig. 1b

Fig. 1c

Fig. 2a

Pantothenate

Fig. 2b

Protamine

Fig. 2c

Methionine

Fig. 3

Fig. 4

Fig. 5a

Fig. 5b

Fig. 6a

Fig. 6b

## INTERNATIONAL SEARCH REPORT

| International application No. |
|---|
| **PCT/JP2024/019454** |

### A. CLASSIFICATION OF SUBJECT MATTER

*A61K 31/16*(2006.01)i; *A23L 33/10*(2016.01)i; *A23L 33/15*(2016.01)i; *A23L 33/17*(2016.01)i; *A23L 33/18*(2016.01)i; *A23L 33/105*(2016.01)i; *A23L 33/115*(2016.01)i; *A23L 33/175*(2016.01)i; *A61K 8/42*(2006.01)i; *A61K 8/44*(2006.01)i; *A61K 8/46*(2006.01)i; *A61K 8/64*(2006.01)i; *A61K 8/9789*(2017.01)i; *A61K 8/9794*(2017.01)i; *A61K 31/047*(2006.01)i; *A61K 31/197*(2006.01)i; *A61K 31/198*(2006.01)i; *A61K 31/7032*(2006.01)i; *A61K 36/28*(2006.01)i; *A61K 36/46*(2006.01)i; *A61K 36/53*(2006.01)i; *A61K 36/54*(2006.01)i; *A61K 36/63*(2006.01)i; *A61K 36/64*(2006.01)i; *A61K 36/65*(2006.01)i; *A61K 36/82*(2006.01)i; *A61K 36/258*(2006.01)i; *A61K 36/355*(2006.01)i; *A61K 36/899*(2006.01)i; *A61K 36/9068*(2006.01)i; *A61K 38/17*(2006.01)i; *A61P 1/02*(2006.01)i; *A61P 3/00*(2006.01)i; *A61P 3/04*(2006.01)i; *A61P 3/10*(2006.01)i; *A61P 9/00*(2006.01)i; *A61P 13/12*(2006.01)i; *A61P 19/10*(2006.01)i; *A61P 21/00*(2006.01)i; *A61P 25/00*(2006.01)i; *A61P 25/28*(2006.01)i; *A61P 27/02*(2006.01)i; *A61P 43/00*(2006.01)i; *A61Q 11/00*(2006.01)i; *C07K 14/46*(2006.01)i

FI: A61K31/16; A23L33/10; A23L33/105; A23L33/115; A23L33/15; A23L33/17; A23L33/175; A23L33/18; A61K8/42; A61K8/44; A61K8/46; A61K8/64; A61K8/9789; A61K8/9794; A61K31/047; A61K31/197; A61K31/198; A61K31/7032; A61K36/258; A61K36/28; A61K36/355; A61K36/46; A61K36/53; A61K36/54; A61K36/63; A61K36/64; A61K36/65; A61K36/82; A61K36/899; A61K36/9068; A61K38/17; A61P1/02; A61P3/00; A61P3/04; A61P3/10; A61P9/00; A61P13/12; A61P19/10; A61P21/00; A61P25/00; A61P25/28; A61P27/02; A61P43/00 107; A61P43/00 111; A61P43/00 121; A61Q11/00; C07K14/46

According to International Patent Classification (IPC) or to both national classification and IPC

### B. FIELDS SEARCHED

Minimum documentation searched (classification system followed by classification symbols)

A61K31/16; A23L33/10; A23L33/105; A23L33/115; A23L33/15; A23L33/17; A23L33/175; A23L33/18; A61K8/42; A61K8/44; A61K8/46; A61K8/64; A61K8/9789; A61K8/9794; A61K31/047; A61K31/197; A61K31/198; A61K31/7032; A61K36/258; A61K36/28; A61K36/355; A61K36/46; A61K36/53; A61K36/54; A61K36/63; A61K36/64; A61K36/65; A61K36/82; A61K36/899; A61K36/9068; A61K38/17; A61P1/02; A61P3/00; A61P3/04; A61P3/10; A61P9/00; A61P13/12; A61P19/10; A61P21/00; A61P25/00; A61P25/28; A61P27/02; A61P43/00; A61Q11/00; C07K14/46

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY/MEDLINE/EMBASE/BIOSIS (STN)JSTPlus/JMEDPlus/JST7580 (JDreamIII)

---

☑ Further documents are listed in the continuation of Box C.       ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **23 July 2024** | **06 August 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

## INTERNATIONAL SEARCH REPORT

| International application No. |
| --- |
| **PCT/JP2024/019454** |

### C.    DOCUMENTS CONSIDERED TO BE RELEVANT

| Category* | Citation of document, with indication, where appropriate, of the relevant passages | Relevant to claim No. |
| --- | --- | --- |
| X | SLYSHENKOV, Vyacheslav S. et al. Pantothenic acid and pantothenol increase biosynthesis of glutathione by boosting cell energetics. FEBS Letters. 2004, vol. 569, pp. 169-172 abstract, tables 2-4 | 1-15 |
| X | JP 2016-185915 A (SUNSTAR INC.) 27 October 2016 (2016-10-27) claims 1-3, examples 1-6, treatment examples 1-11, paragraph [0013] | 1-15 |
| X | JP 1999-243910 A (SUNSTAR INC.) 14 September 1999 (1999-09-14) claims 1-3, paragraph [0005] | 1-15 |
| X | JP 2009-007292 A (LION CORPORATION) 15 January 2009 (2009-01-15) claim 1, examples 1-15, paragraph [0013] | 1-15 |
| X | JP 2021-530516 A (3M INNOVATIVE PROPERTIES COMPANY) 11 November 2021 (2021-11-11) claims 1-15, embodiments 1-7, paragraph [0003] | 1-15 |
| X | JP 2001-158735 A (SNOW BRAND MILK PRODUCTS CO., LTD.) 12 June 2001 (2001-06-12) claims 1-3, reference example 4, table 1, paragraph [0003] | 1-15 |
| X | JP 2004-196672 A (NIIGATA PREFECTURE) 15 July 2004 (2004-07-15) claims 1-4, examples 1-3, test examples | 1-15 |
| X | JP 2006-328002 A (HIGASHI NOEN K.K.) 07 December 2006 (2006-12-07) claims 1-4, paragraph [0012] | 1-15 |
| X | JP 2022-103980 A (SUNSTAR SUISSE SA) 08 July 2022 (2022-07-08) claims 1-3, examples 2, 14-20, paragraph [0020] | 1-15 |

Form PCT/ISA/210 (second sheet) (July 2022)

## INTERNATIONAL SEARCH REPORT

International application No.

**PCT/JP2024/019454**

| Box No. III | Observations where unity of invention is lacking (Continuation of item 3 of first sheet) |

This International Searching Authority found multiple inventions in this international application, as follows:

Claims 1, 5, and 9 of the present application disclose at least one compound "selected from the group consisting of panthenol, pantothenic acid or a salt thereof, protamine or a salt thereof, and methionine or a salt thereof." However, since panthenol, pantothenic acid, protamine, and methionine is well-known prior to the priority date of the present application that it is not necessary to cite documents, there are no special technical features identical or corresponding to the invention in claim 1 of the present application (between the compounds of panthenol, pantothenic acid, and protamine). Therefore, the invention in claims 1, 5, and 9 of the present application, and claims 2-4, 6-8, and 10-15 referring to any of claims 1, 5, and 9 are classified into the following three inventions.

(Invention 1)
Invention of panthenol, and pantothenic acid or a salt thereof in claims 1-15
(Invention 2)
Invention of protamine or a salt thereof in claims 1-15
(Invention 3)
Invention of methionine or a salt thereof in claims 1-15

1. ☐ As all required additional search fees were timely paid by the applicant, this international search report covers all searchable claims.

2. ☑ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**
☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (July 2022)

## INTERNATIONAL SEARCH REPORT
### Information on patent family members

International application No.

**PCT/JP2024/019454**

| Patent document cited in search report | | | Publication date (day/month/year) | Patent family member(s) | | | Publication date (day/month/year) |
|---|---|---|---|---|---|---|---|
| JP | 2016-185915 | A | 27 October 2016 | (Family: none) | | | |
| JP | 1999-243910 | A | 14 September 1999 | WO | 1999/044440 | A1 | |
| | | | | claims 1-3, p. 2, line 18 to p. 3, line 13 | | | |
| JP | 2009-007292 | A | 15 January 2009 | (Family: none) | | | |
| JP | 2021-530516 | A | 11 November 2021 | WO | 2020/016713 | A1 | |
| | | | | claims 1-15, embodiments 1-7, p. 1, lines 14-17 | | | |
| | | | | US | 2021/0121380 | A1 | |
| | | | | EP | 3823586 | A1 | |
| | | | | CN | 112533579 | A | |
| JP | 2001-158735 | A | 12 June 2001 | (Family: none) | | | |
| JP | 2004-196672 | A | 15 July 2004 | (Family: none) | | | |
| JP | 2006-328002 | A | 07 December 2006 | (Family: none) | | | |
| JP | 2022-103980 | A | 08 July 2022 | (Family: none) | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *J Clin Periodontol*, 2017, vol. 44, 463-471